(19) Europäisches Patentamt
European Patent Office
Office européen des brevets

(11) Veröffentlichungsnummer: **0 010 163**
**B1**

# (12) EUROPÄISCHE PATENTSCHRIFT

(45) Veröffentlichungstag der Patentschrift: **06.01.82**

(51) Int. Cl.³: **C 07 D 271/06,** C 07 D 285/08, A 01 N 43/82

(21) Anmeldenummer: **79103468.9**

(22) Anmeldetag: **17.09.79**

(54) **N-Diazolylalkyl-chloracetanilide, Verfahren zu ihrer Herstellung sowie ihre Verwendung als Herbizide.**

(30) Priorität: **28.09.78 DE 2842284**

(43) Veröffentlichungstag der Anmeldung: **30.04.80 Patentblatt 80/9**

(45) Bekanntmachung des Hinweises auf die Patenterteilung: **06.01.82 Patentblatt 82/1**

(84) Benannte Vertragsstaaten: **AT BE CH DE FR GB IT NL**

(56) Entgegenhaltungen:
**DE-A-2 648 008**
**DE-A-2 704 281**
**DE-A-2 742 583**
**EP-A-0 003 540**

(73) Patentinhaber: **BAYER AG, Zentralbereich Patente, Marken und Lizenzen, D-5090 Leverkusen 1, Bayerwerk (DE)**

(72) Erfinder: **Stetter, Jörg, Dr., Pahlkestrasse 3, D-5600 Wuppertal 1 (DE)**
Erfinder: **Ditgens, Klaus, Dr., Claudiusweg 7, D-5600 Wuppertal 1 (DE)**
Erfinder: **Thomas, Rudolf, Dr., Wilkhausstrasse 129, D-5600 Wuppertal 2 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

EP 0 010 163 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# N-Diazolylalkyl-chloracetanilide, Verfahren zu ihrer Herstellung sowie ihre Verwendung als Herbizide

Die vorliegende Erfindung betrifft neue N-Diazolylalkylchloracetanilide, mehrere Verfahren zu ihrer Herstellung sowie ihre Verwendung als Herbizide.

Es ist bereits bekanntgeworden, daß man 2,6-Diethyl-N-methoxymethyl-chloracetanilid zur selektiven Unkrautbekämpfung verwenden kann (vgl. R. Wegler, Chemie der Pflanzenschutz- und Schädlingsbekämpfungsmittel, Band 5, Seite 255, Springer-Verlag (1977)). Diese Verbindung ist jedoch nicht immer ausreichend wirksam und in ihrer Selektivität nicht immer ganz befriedigend.

Weiterhin ist bereits bekanntgeworden, daß zahlreiche N-substituierte Halogenacetanilide, in denen das Stickstoffatom des Anilinteiles über eine $CH_2$-Gruppe mit einem gegebenenfalls substituierten Azol über ein N-Atom des Azol-Restes verbunden ist, gute herbizide Eigenschaften besitzen (vgl. DE-A-2 704 281 und DE-A-2 742 583). Die selektive herbizide Wirksamkeit dieser Stoffe läßt jedoch in manchen Fällen zu wünschen übrig.

Es wurden nun neue N-Diazolylalkyl-chloracetanilide der Formel

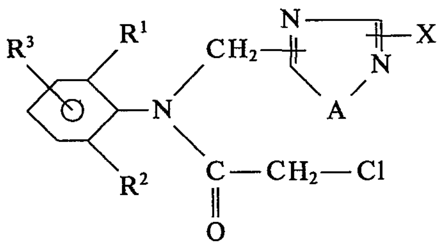

(I)

in welcher

A für Sauerstoff oder Schwefel steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
X für Wasserstoff, Alkyl mit 1 bis 4 Kohlenstoffatomen, Alkoxy mit 1 bis 4 Kohlenstoffatomen, Alkylthio mit 1 bis 4 Kohlenstoffatomen, Phenyl oder Phenoxy steht und

der Diazolyl-Rest über ein Kohlenstoffatom gebunden ist,
gefunden.

Weiterhin wurde gefunden, daß man die N-Diazolylalkyl-chloracetanilide der Formel (I) erhält, wenn man

a) N-Diazolylalkyl-aniline der Formel

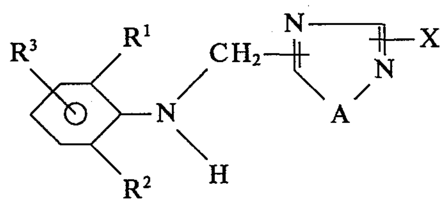

(II)

in welcher
A, $R^1$ $R^2$, $R^3$ und X die oben angegebene Bedeutung haben,

und der Diazolyl-Rest über ein Kohlenstoffatom gebunden ist, mit Chloressigsäurechlorid oder -bromid bzw. -anhydrid der Formeln

$$Cl—CH_2—CO—Cl(Br) \qquad (IIIa)$$

bzw.

$$(Cl—CH_2—CO)_2O \qquad (IIIb)$$

in Gegenwart eines Verdünnungsmittels und gegebenenfalls in Gegenwart eines Säurebindemittels umsetzt, oder

b) Chloracetanilide der Formel

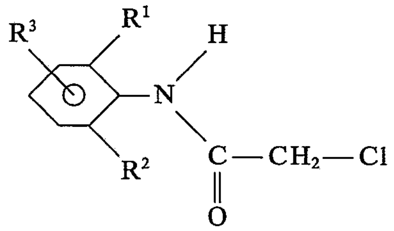

(IV)

in welcher
$R^1$, $R^2$ und $R^3$ die oben angegebene Bedeutung haben,

mit Diazolylalkyl-Derivaten der Formel

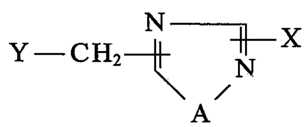

(V)

in welcher

A und X die oben angegebene Bedeutung haben und
Y für Halogen, den Mesylat- oder Tosylat-Rest steht, und der Diazolyl-Rest über ein Kohlenstoffatom gebunden ist,

in Gegenwart eines Säurebinders und gegebenenfalls in Gegenwart eines organischen Verdünnungsmittels umsetzt.

Die neuen N-Diazolylalkyl-chloracetanilide der Formel (I) weisen starke herbizide, insbesondere auch selektiv-herbizide Eigenschaften auf.

Überraschenderweise zeigen die erfindungsgemäßen N-Diazolylalkyl-chloracetanilide bei sehr guter Unkrautwirkung bessere Möglichkeiten zum Einsatz als selektive Unkrautbekämpfungsmittel in wichtigen Kulturpflanzen als das aus dem Stand der Technik bekannte 2,6-Diethyl-N-methoxymethyl-chloracetanilid, welches ein hochaktiver Wirkstoff gleicher Wirkungsart ist.

Ferner übertreffen die erfindungsgemäßen N-Diazolylalkyl-chloracetanilide auch konstitutionell ähnliche Stoffe, die in der DE-A-2 704 281 bzw. in der DE-A-2 742 583 offenbart werden, bezüglich ihrer selektiven herbiziden Wirksamkeit. So läßt sich das erfindungsgemäße 2,6-Diethyl-N-(3′-methyl-1′,2′,4′-oxadiazol-5′-yl-methyl)-chlor-acetanilid besser zur selektiven Unkrautbekämpfung, z. B. in Baumwolle, Mais und Rüben einsetzen als die vorbekannten Verbindungen
2,6-Diethyl-N-(3-chlor-1,2,4-triazol-1-yl-methyl)-chloracetanilid,
2,6-Diethyl-N-(1,2,4-triazol-1-yl-methyl)-chloracetanilid,
2-Methyl-6-ethyl-N-(1,2,4-triazol-1-yl-methyl)-chloracetanilid,
2-tert.-Butyl-N-(1,2,4-triazol-1-yl-methyl)-chloracetanilid und
2,6-Dimethyl-N-(1,2,4-triazol-1-yl-methyl)-chloracetanilid.

Die erfindungsgemäße Verwendung gemäß Beispiel 4 besitzt eine stärkere Unkrautwirkung als die zum Vergleich herangezogenen bekannten Verbindungen und ist darüber hinaus auch sehr gut verträglich bei Baumwolle und Rüben.

Die erfindungsgemäßen N-Diazolylalkyl-chloracetanilide sind durch die Formel (I) allgemein definiert. Besonders bevorzugt sind diejenigen Stoffe, in denen A für Sauerstoff oder Schwefel steht; $R^1$ für Methyl, Ethyl, Isopropyl, sek.-Butyl oder tert.-Butyl steht; $R^2$ für Wasserstoff, Methyl, Ethyl, Isopropyl, sek.-Butyl oder tert.-Butyl steht; $R^3$ für Wasserstoff, Methyl, Ethyl, Isopropyl, sek.-Butyl oder tert.-Butyl steht und X für Wasserstoff, Methyl, Ethyl, n-Propyl, Isopropyl, n-Butyl, sek.-Butyl, tert.-Butyl, Methoxy, Ethoxy, Isopropoxy, Methylthio, Ethylthio, Isopropylthio, Phenyl oder Phenoxy steht.

Im einzelnen seien außer den bei den Herstellungsbeispielen genannten Verbindungen die folgenden Verbindungen der allgemeinen Formel (I) genannt:

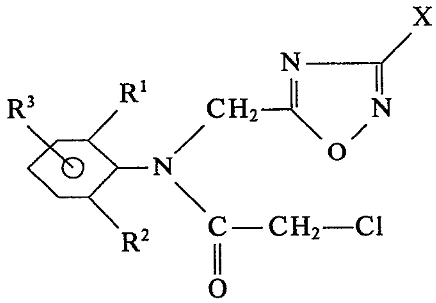

(Ia)

| $R^1$ | $R^2$ | $R^3$ | X |
|---|---|---|---|
| $CH_3$ | $CH_3$ | H | $CH_3$ |
| $C(CH_3)_3$ | H | H | $CH_3$ |
| $CH_3$ | H | 3-$CH_3$ | $CH_3$ |
| $CH_3$ | H | 5-$CH_3$ | $CH_3$ |
| $C_2H_5$ | $C_2H_5$ | H | H |
| $CH_3$ | $C_2H_5$ | H | H |
| $CH_3$ | $CH_3$ | H | H |
| $C(CH_3)_3$ | H | H | H |
| $CH_3$ | H | 3-$CH_3$ | H |
| $CH_3$ | H | 5-$CH_3$ | H |
| $C_2H_5$ | $CH_3$ | H | $C_2H_5$ |
| $C_2H_5$ | $C_2H_5$ | H | $C_2H_5$ |
| $CH_3$ | $CH_3$ | H | $C_2H_5$ |
| $C_2H_5$ | $CH_3$ | H | 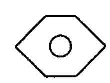 |
| $C_2H_5$ | $C_2H_5$ | H | 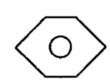 |
| $CH_3$ | $CH_3$ | H | 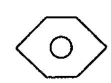 |
| $C_2H_5$ | $CH_3$ | H | $C_3H_7$ |
| $C_2H_5$ | $C_2H_5$ | H | $C_3H_7$ |
| $CH_3$ | $CH_3$ | H | $C_3H_7$ |
| $C_2H_5$ | $CH_3$ | H | $OCH_3$ |
| $C_2H_5$ | $C_2H_5$ | H | $OCH_3$ |
| $CH_3$ | $CH_3$ | H | $OCH_3$ |

Fortsetzung

| $R^1$ | $R^2$ | $R^3$ | X |
|---|---|---|---|
| $C_2H_5$ | $CH_3$ | H | $SCH_3$ |
| $C_2H_5$ | $C_2H_5$ | H | $SCH_3$ |
| $CH_3$ | $CH_3$ | H | $SCH_3$ |

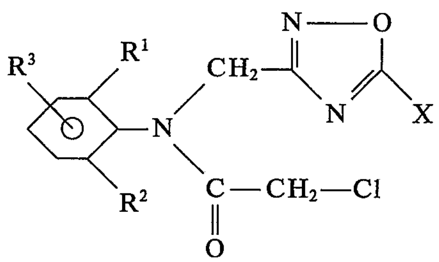

(Ib)

| $R^1$ | $R^2$ | $R^3$ | X |
|---|---|---|---|
| $CH_3$ | $CH_3$ | H | $CH_3$ |
| $C(CH_3)_3$ | H | H | $CH_3$ |
| $CH_3$ | H | $3\text{-}CH_3$ | $CH_3$ |
| $CH_3$ | H | $5\text{-}CH_3$ | $CH_3$ |
| $C_2H_5$ | $C_2H_5$ | H | H |
| $CH_3$ | $C_2H_5$ | H | H |
| $CH_3$ | $CH_3$ | H | H |
| $C(CH_3)_3$ | H | H | H |
| $CH_3$ | H | $3\text{-}CH_3$ | H |
| $CH_3$ | H | $5\text{-}CH_3$ | H |
| $CH_3$ | $C_2H_5$ | H | $C_2H_5$ |
| $CH_3$ | $CH_3$ | H | $C_2H_5$ |
| $C_2H_5$ | $C_2H_5$ | H | $n\text{-}C_3H_7$ |
| $CH_3$ | $C_2H_5$ | H | $n\text{-}C_3H_7$ |
| $CH_3$ | $CH_3$ | H | $n\text{-}C_3H_7$ |
| $CH_3$ | $C_2H_5$ | H | $i\text{-}C_3H_7$ |
| $C_2H_5$ | $C_2H_5$ | H | $i\text{-}C_3H_7$ |
| $CH_3$ | $CH_3$ | H | $i\text{-}C_3H_7$ |

Fortsetzung

| $R^1$ | $R^2$ | $R^3$ | X |
|---|---|---|---|
| $CH_3$ | $C_2H_5$ | H | n-$C_4H_9$ |
| $C_2H_5$ | $C_2H_5$ | H | n-$C_4H_9$ |
| $CH_3$ | $CH_3$ | H | n-$C_4H_9$ |
| $CH_3$ | $C_2H_5$ | H | t-$C_4H_9$ |
| $C_2H_5$ | $C_2H_5$ | H | t-$C_4H_9$ |
| $CH_3$ | $CH_3$ | H | t-$C_4H_9$ |
| $C_2H_5$ | $C_2H_5$ | H | 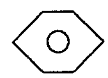 |
| $CH_3$ | $C_2H_5$ | H | 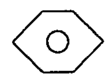 |
| $CH_3$ | $CH_3$ | H | 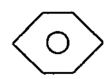 |
| $C_2H_5$ | $C_2H_5$ | H | $OCH_3$ |
| $CH_3$ | $C_2H_5$ | H | $OCH_3$ |
| $C_2H_5$ | $C_2H_5$ | H | $OC_2H_5$ |
| $CH_3$ | $C_2H_5$ | H | $OC_2H_5$ |
| $C_2H_5$ | $C_2H_5$ | H | 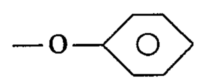 |
| $CH_3$ | $C_2H_5$ | H | 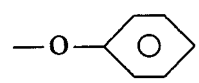 |

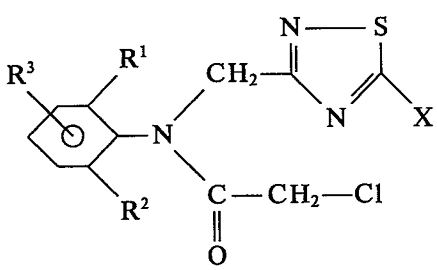

(Ic)

| $R^1$ | $R^2$ | $R^3$ | X |
|---|---|---|---|
| $CH_3$ | $CH_3$ | H | H |
| $CH_3$ | $C_2H_5$ | H | H |
| $C_2H_5$ | $C_2H_5$ | H | H |
| $CH_3$ | H | 3-$CH_3$ | H |

Fortsetzung

| $R^1$ | $R^2$ | $R^3$ | X |
|---|---|---|---|
| $CH_3$ | H | $5\text{-}CH_3$ | H |
| $CH_3$ | $CH_3$ | H | $CH_3$ |
| $CH_3$ | $C_2H_5$ | H | $CH_3$ |
| $C_2H_5$ | $C_2H_5$ | H | $CH_3$ |
| $C(CH_3)_3$ | H | H | $CH_3$ |
| $CH_3$ | $C_2H_5$ | H | 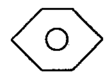 |
| $C_2H_5$ | $C_2H_5$ | H | 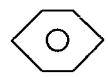 |
| $CH_3$ | $CH_3$ | H | $OCH_3$ |
| $CH_3$ | $C_2H_5$ | H | $OCH_3$ |
| $C_2H_5$ | $C_2H_5$ | H | $OCH_3$ |
| $C(CH_3)_3$ | H | H | $OCH_3$ |
| $CH_3$ | $CH_3$ | H | $SCH_3$ |
| $CH_3$ | $C_2H_5$ | H | $SCH_3$ |
| $C_2H_5$ | $C_2H_5$ | H | $SCH_3$ |
| $C(CH_3)_3$ | H | H | $SCH_3$ |
| $CH_3$ | $C_2H_5$ | H | $OC_2H_5$ |
| $C_2H_5$ | $C_2H_5$ | H | $OC_2H_5$ |
| $CH_3$ | $C_2H_5$ | H | 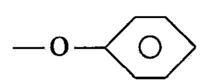 |
| $C_2H_5$ | $C_2H_5$ | H | 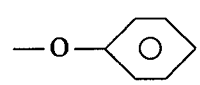 |
| $CH_3$ | $C_2H_5$ | H | $SC_2H_5$ |
| $C_2H_5$ | $C_2H_5$ | H | $SC_2H_5$ |

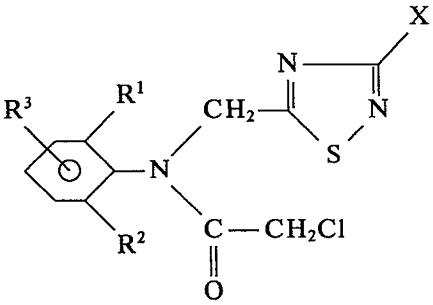

(Id)

| $R^1$ | $R^2$ | $R^3$ | X |
|---|---|---|---|
| $CH_3$ | $CH_3$ | H | H |
| $CH_3$ | $C_2H_5$ | H | H |
| $C_2H_5$ | $C_2H_5$ | H | H |
| $CH_3$ | H | 3-$CH_3$ | H |
| $CH_3$ | H | 5-$CH_3$ | H |
| $CH_3$ | $CH_3$ | H | $CH_3$ |
| $CH_3$ | $C_2H_5$ | H | $CH_3$ |
| $C_2H_5$ | $C_2H_5$ | H | $CH_3$ |
| $C(CH_3)_3$ | H | H | $CH_3$ |
| $CH_3$ | $C_2H_5$ | H | 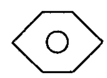 |
| $C_2H_5$ | $C_2H_5$ | H | 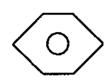 |
| $CH_3$ | $CH_3$ | H | $OCH_3$ |
| $CH_3$ | $C_2H_5$ | H | $OCH_3$ |
| $C_2H_5$ | $C_2H_5$ | H | $OCH_3$ |
| $C(CH_3)_3$ | H | H | $OCH_3$ |
| $CH_3$ | $CH_3$ | H | $SCH_3$ |
| $C_2H_5$ | $C_2H_5$ | H | $SCH_3$ |
| $C(CH_3)_3$ | H | H | $SCH_3$ |
| $CH_3$ | $C_2H_5$ | H | $OC_2H_5$ |
| $C_2H_5$ | $C_2H_5$ | H | $OC_2H_5$ |
| $CH_3$ | $C_2H_5$ | H | 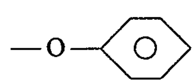 |
| $C_2H_5$ | $C_2H_5$ | H | 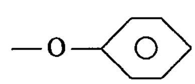 |

Fortsetzung

| $R^1$ | $R^2$ | $R^3$ | X |
|---|---|---|---|
| $CH_3$ | $C_2H_5$ | H | $SC_2H_5$ |
| $C_2H_5$ | $C_2H_5$ | H | $SC_2H_5$ |
| $CH_3$ | $C_2H_5$ | H | $SCH_3$ |

Verwendet man 2-Ethyl-6-methyl-N-(5'-methyl-1',2',4'-oxadiazol-3'-yl-methyl)-anilin und Chloracetylchlorid als Ausgangsstoffe, so kann der Reaktionsablauf durch das folgende Formelschema wiedergegeben werden (Verfahrensvariante a):

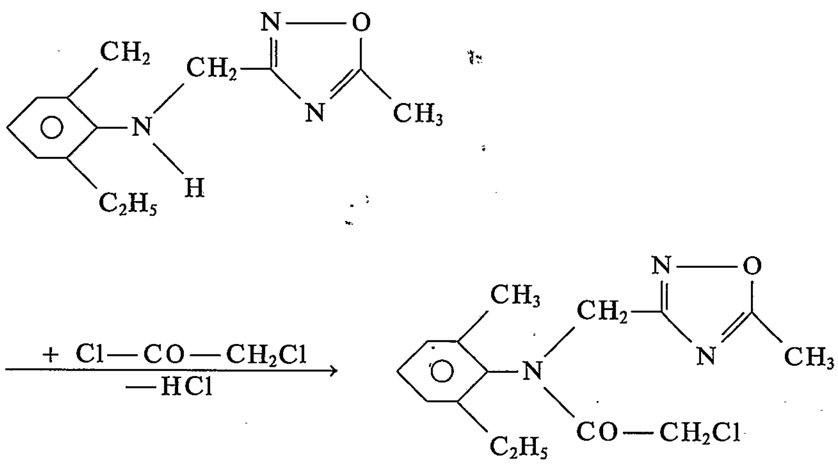


Verwendet man 2-Ethyl-6-methyl-chloracetanilid und 5-Brom-methyl-3-methyl-1,2,4-oxadiazol als Ausgangsstoffe, so kann der Reaktionsablauf durch das folgende Formelschema wiedergegeben werden (Verfahrensvariante b):

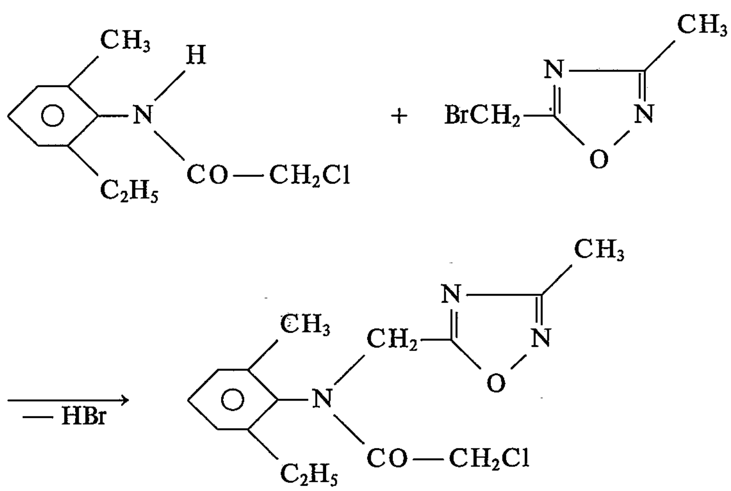


Die bei der Durchführung des erfindungsgemäßen Verfahrens (a) als Ausgangsstoffe benötigten N-Diazolylalkylaniline sind durch die Formel (II) allgemein definiert. In dieser Formel stehen A, $R^1$, $R^2$, $R^3$ und X für diejenigen Reste, die bereits im Zusammenhang mit der Beschreibung der erfindungsgemäßen Stoffe der Formel (I) für diese Reste genannt wurden.

Die N-Diazolylalkyl-aniline der Formel (II) sind noch nicht bekannt. Man erhält sie, wenn man Aniline der Formel

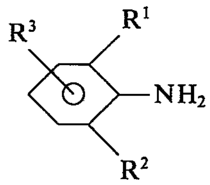

(VI)

in welcher
$R^1$, $R^2$ und $R^3$ die oben angegebene Bedeutung haben,

mit Diazolylalkyl-Derivaten der Formel

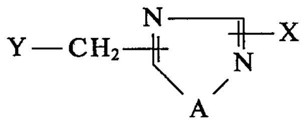

(V)

in welcher
A, X und Y die oben angegebene Bedeutung haben, und der Diazolyl-Rest über ein Kohlenstoffatom gebunden ist,

in Gegenwart eines Säurebinders und gegebenenfalls in Gegenwart eines Verdünnungsmittels umsetzt.

Die bei der Herstellung der N-Diazolylalkyl-aniline der Formel (II) als Ausgangsstoffe benötigten Aniline der Formel (VI) sind allgemein bekannte Verbindungen der organischen Chemie. Als Beispiele seien genannt:

Anilin; 2-Methylanilin; 2-Ethylanilin; 2-Isopropylanilin; 2-sek.-Butylanilin; 2-tert.-Butylanilin; 2,6-Dimethylanilin; 2,3-Dimethylanilin; 2,5-Dimethylanilin; 2,6-Diethylanilin; 2-Ethyl-6-methylanilin; 2,4,6-Trimethylanilin; 2-Ethyl-4,6-dimethylanilin; 2,6-Diethyl-4-methyl-anilin; 2,6-Diisopropyl-4-methyl-anilin; 2,3,6-Trimethylanilin.

Bei der Herstellung der N-Diazolylalkyl-aniline der Formel (II) können als Säurebinder alle üblichen Säureakzeptoren verwendet werden. Vorzugsweise in Betracht kommen Alkalicarbonate wie Kalium- oder Natriumcarbonat.

Als Verdünnungsmittel können bei der Herstellung der N-Diazolylalkyl-aniline der Formel (II) alle üblichen inerten organischen Lösungsmittel eingesetzt werden. Vorzugsweise in Betracht kommen Dimethylformamid und Toluol.

Die Reaktionstemperaturen können bei der Herstellung der N-Diazolylalkyl-aniline der Formel (II) nach dem obigen Verfahren innerhalb eines größeren Bereiches variiert werden. Im allgemeinen arbeitet man zwischen 0° C und 180° C, orzugsweise zwischen 20° C und 160° C.

Bei der Herstellung der N-Diazolylalkyl-aniline der Formel (II) nach dem obigen Verfahren setzt man die Aniline der Formel (VI) und die Diazolylalkyl-Derivate der Formel (V) im allgemeinen in äquimolaren Mengen ein. Es ist jedoch auch möglich, eine der Komponenten, vorzugsweise das Anilin der Formel (VI), in einem Überschuß einzusetzen. — Die Aufarbeitung und Isolierung der Reaktionsprodukte erfolgt nach üblichen Methoden (vgl. auch die Herstellungsbeispiele).

Die außerdem für das erfindungsgemäße Verfahren (a) als Ausgangsstoffe zu verwendenden Stoffe Chloressigsäurechlorid und -bromid bzw. -anhydrid sind durch die Formeln (IIIa) und (IIIb) definiert.

Die bei der Durchführung des erfindungsgemäßen Verfahrens (b) als Ausgangsstoffe benötigten Chloracetanilide sind durch die Formel (IV) allgemein definiert. In dieser Formel stehen $R^1$, $R^2$ und $R^3$ für diejenigen Reste, die bereits im Zusammenhang mit der Beschreibung der erfindungsgemäßen Stoffe der Formel (I) für die Reste genannt wurden.

Die Halogenacetanilide der Formel (IV) sind allgemein bekannt bzw. können sie in allgemein bekannter Art und Weise erhalten werden, indem man entsprechende Aniline mit Chloressigsäurechlorid oder -bromid bzw. -anhydrid der Formeln (IIIa) bzw. (IIIb) in Gegenwart eines inerten organischen Lösungsmittels, wie beispielsweise Toluol oder Dimethylformamid, gegebenenfalls in Gegenwart eines Säurebindemittels, wie z. B. Kaliumcarbonat oder Triethylamin, bei Temperaturen zwischen 0° C und 100° C umsetzt (vergleiche auch die Herstellungsbeispiele).

Die außerdem für das erfindungsgemäße Verfahren (b) als Ausgangsstoffe zu verwendenden Diazolylalkyl-Derivate sind durch die Formel (V) allgemein definiert. In dieser Formel stehen A und X für diejenigen Reste, die bereits im Zusammenhang mit der Beschreibung der erfindungsgemäßen Stoffe der Formel (I) für diese Reste genannt wurden. Y steht vorzugsweise für Chlor, Brom, den Mesylat- und Tosylat-Rest.

Die Diazolyl-Derivate der Formel (V) sind bekannt (vergleiche u. a. Deutsche Offenlegungsschrift 19 15 495 und 20 54 342, die US-Patentschriften 3 211 742 und 3 264 318 sowie Bull. Soc. Chim. Belges 73, (1964) 793); bzw. können sie nach den dort beschriebenen Verfahren in allgemein bekannter Art und Weise erhalten werden.

Als Verdünnungsmittel kommen für die erfindungsgemäße Umsetzung gemäß Verfahrensvariante (a) vorzugsweise inerte organische Lösungsmittel infrage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid Tetrachlorkohlenstoff, Chloroform oder Chlorbenzol; und Ester, wie Essigester.

Das erfindungsgemäße Verfahren (a) kann gegebenenfalls in Gegenwart von Säurebindern (Chlorwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0° C und 120° C, vorzugsweise zwischen 20° C und 100° C.

Bei der Durchführung des erfindungsgemäßen Verfahrens (a) setzt man vorzugsweise auf 1 Mol der Verbindung der Formel (II) 1 bis 1,5 Mol Chloracetylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) alle inerten, mit Wasser nicht mischbaren, organischen Lösungsmittel infrage. Hierzu gehören vorzugsweise Ether, wie Diethylether; aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Die erfindungsgemäße Umsetzung gemäß Verfahren (b) wird in Gegenwart eines Säurebindemittels durchgeführt. Als solche können alle üblichen säurebindemittel verwendet werden. Hierzu gehören vorzugsweise anorganische Basen, wie beispielsweise Alkalihydroxide und Alkalicarbonate.

Die Reaktionstemperaturen können bei der Durchführung des erfindungsgemäßen Verfahrens (b) in einem größeren Bereich variiert werden. Im allgemeinen arbeitet man zwischen −70° C und +100° C, vorzugsweise zwischen −20° C und +80° C.

Bei der Durchführung des erfindungsgemäßen Verfahrens (b) setzt man vorzugsweise auf 1 Mol Chloracetanilid der Formel (IV) 1 bis 1,5 Mol Diazolyl-alkyl-Derivat der Formel (V) ein. Die Isolierung der Verbindungen der Formel (I) erfolgt in üblicher Weise.

In einer bevorzugten Ausführungsform wird die erfindungsgemäße Umsetzung gemäß Verfahren (b) in einem Zweiphasensystem, wie beispielsweise wäßrige Natron- oder Kalilauge/Toluol oder Methylenchlorid, gegebenenfalls unter Zusatz von 0,1 — 1 Mol eines Phasen-Transfer-Katalysators, wie beispielsweise Ammonium- oder Phosphoniumverbindungen, beispielsweise seien Benzyl-dodecyldimethyl-ammonium-chlorid (Zephirol) und Triethyl-benzylammoniumchlorid genannt, durchgeführt (vergleiche auch die Herstellungsbeispiele).

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forts-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen insbesondere neben einer sehr guten Wirkung gegen grasartige Unkräuter auch eine gute herbizide Wirkung bei breitblättrigen Unkräutern. Ein selektiver Einsatz der erfindungsgemäßen Wirkstoff ist möglich, vorzugsweise in Mais, Erdnüssen, Rüben, Sojabohnen, Baumwolle, Reis und andere Getreidearten.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinverkapselungen in polymeren Stoffen.

Diese Formulierungen werden in bekannter Weise hergestellt, z. B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylakohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

In dem nachfolgenden Beispiel werden die nachstehend angegebenen Verbindungen als Vergleichssubstanzen eingesetzt:

A = 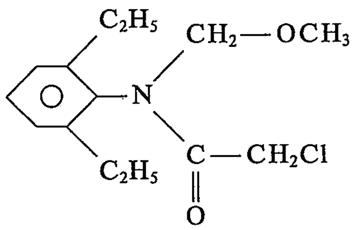

(2,6-Diethyl-N-methoxymethyl-chloracetanilid).

(B) = 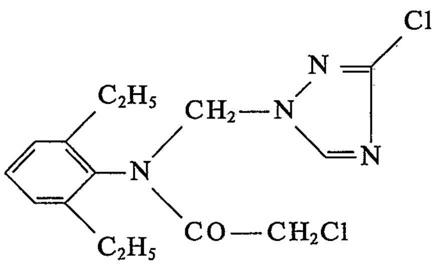

(bekannt aus DE-A-2 742 583)

(C) = 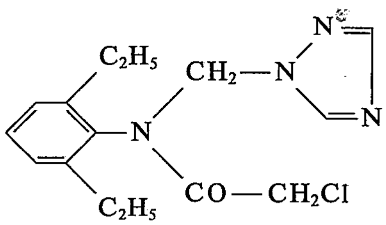

(bekannt aus DE-A-2 704 281)

(D) = 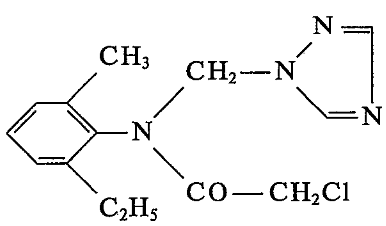

(bekannt aus DE-A-2 704 281)

(E) = 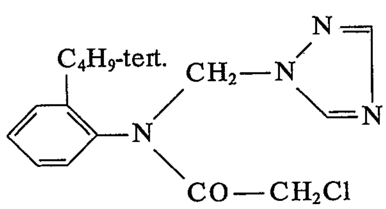

(bekannt aus DE-A-2 742 583)

(F) = 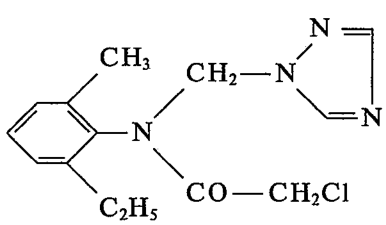

(bekannt aus DE-A-2 742 583)

## Beispiel A

## Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate werden ermittelt.

In diesem Test zeigen die erfindungsgemäßen Verbindungen gemäß Beispiel 1, 2, 3 und 4 eine bessere selektive Wirksamkeit als die aus dem Stand der Technik bekannten Verbindungen.

## Herstellungsbeispiele

### Beispiel 1

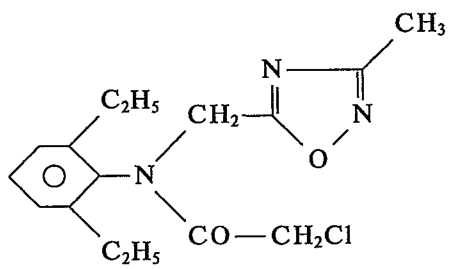

(Verfahren a)

8,7 g (0,035 Mol) 2,6-Diethyl-N-(3′-methyl-1′,2′,4′-oxadiazol-5′-yl-methyl)-anilin und 3 g (0,037 Mol) Pyridin werden in 100 ml trockenem Tetrahydrofuran zum Sieden erhitzt. Unter Rühren werden dann 4,1 g (0,036 Mol) Chloracetylchlorid zugetropft. Nach 15 Minuten wird die gesamte Reaktionsmischung im Vakuum eingeengt und mit Wasser versetzt. Die dabei ausfallenden Kristalle werden abgesaugt und aus wenig Essigester umkristallisiert. Man erhält 9,4 g (83% der Theorie) 2,6-Diethyl-N-(3′-methyl-1′,2′,4′-oxadiazol-5′-yl-methyl)-chloracetanilid vom Schmelzpunkt 70 – 72° C.

Herstellung des Ausgangsproduktes

(II-1)

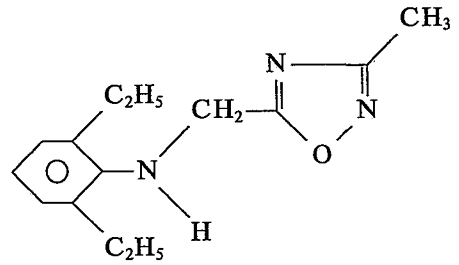

36,8 g (0,25 Mol) 2,6-Diethylanilin, 13,8 g (0,1 Mol) gepulvertes Kaliumcarbonat und 13 g (0,1 Mol) 5-Chlormethyl-3-methyl-1,2,4-oxadiazol werden in 25 ml Dimethylformamid unter Rühren 5 Stunden auf 100° C erhitzt. Danach wird vom anorganischen Salz abgesaugt, das Filtrat auf 100 ml Wasser gegossen und mit Methylenchlorid extrahiert. Die organische Phase wird über Natriumsulfat getrocknet und durch Abdestillieren des Lösungsmittels im Vakuum eingeengt. Der ölige Rückstand wird im Hochvakuum destilliert. Man erhält 12,2 g (50% der Theorie) 2,6-Diethyl-N-(3′-methyl-1′,2′,4′-oxadiazol-5′-yl-methyl)-anilin vom Siedepunkt 145° C/0,05 mm.

Beispiel 2

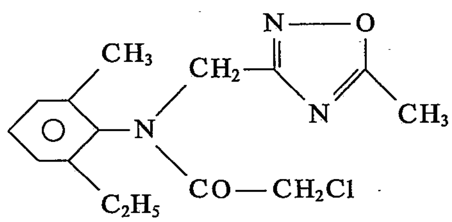

(Verfahren a)

Zu einer siedenden Mischung aus 4,6 g (0,02 Mol) 2-Ethyl-6-methyl-N-(5′-methyl-1′,2′,4′-oxadiazol-3′-yl-methyl)-anilin und 2 g (0,025 Mol) Pyridin in 100 ml trockenem Tetrahydrofuran werden 2,3 g (0,02 Mol) Chloracetylchlorid langsam zugetropft. Nach 15 Minuten wird die Reaktionsmischung im Vakuum eingeengt, mit Wasser versetzt und mit Methylenchlorid extrahiert. Die organische Phase wird über Natriumsulfat getrocknet und durch Abdestillieren des Lösungsmittels im Vakuum eingeengt. Der Rückstand wird durch Anreiben mit Petrolether zur Kristallisation gebracht. Man erhält 5 g (81% der Theorie) 2-Ethyl-6-methyl-N-(5′-methyl-1′,2′,4′-oxadiazol-3′-yl-methyl)-chloracetanilid vom Schmelzpunkt 69 – 72° C.

Herstellung des Ausgangsproduktes

(II-2)

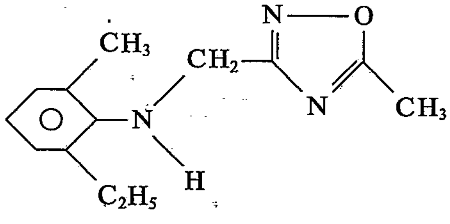

13 g (0,1 Mol) 3-Chlormethyl-5-methyl-1,2,4-oxadiazol werden unter Rühren bei 100° C in eine Mischung von 27 g (0,2 Mol) 2-Ethyl-6-methyl-anilin und 13,8 g (0,1 Mol) Kaliumcarbonat getropft. Man läßt 6 Stunden bei 100° C nachrühren und gießt anschließend die Reaktionsmischung auf Wasser. Man extrahiert mit Methylenchlorid, trocknet die organische Phase über Natriumsulfat und engt durch Abdestillieren des Lösungsmittels im Vakuum ein. Der Rückstand wird im Hochvakuum destilliert. Man erhält 5,2 g (22,5% der Theorie) 2-Ethyl-6-methyl-N-(5′-methyl-1′,2′,4′-oxadiazol-3′-yl-methyl)-anilin vom Siedepunkt 132° C/0,2 mm und einem Schmelzpunkt von 54 – 55° C.

In entsprechender Weise werden diejenigen Verbindungen erhalten, die in der Tabelle 1 formelmäßig aufgeführt sind.

Tabelle 1

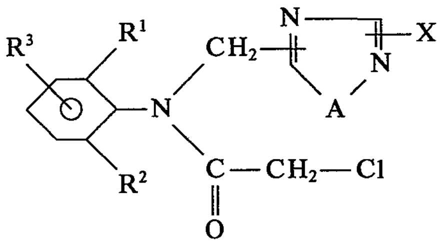

(I)

| Bsp. Nr. | $R^1$ | $R^2$ | $R^3$ | 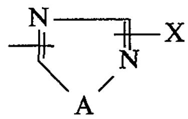 | Schmelzpunkt (°C) bzw. Brechungsindex |
|---|---|---|---|---|---|
| 3 | $CH_3$ | $C_2H_5$ | H | 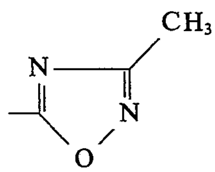 | Öl |
| 4 | $C_2H_5$ | $C_2H_5$ | H | 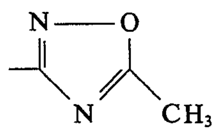 | 91–92 |
| 5 | $C_2H_5$ | $C_2H_5$ | H | 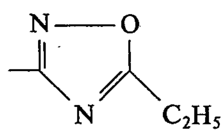 | 39–41 |
| 6 | $CH_3$ | $C_2H_5$ | H | 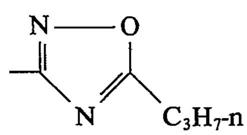 | viskoses Öl |
| 7 | $CH_3$ | $C_2H_5$ | H | 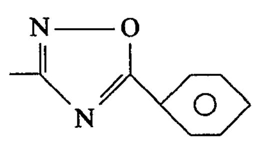 | viskoses Öl |
| 8 | $CH_3$ | $C_2H_5$ | H | 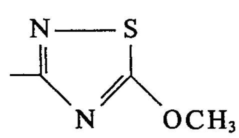 | viskoses Öl |
| 9 | $CH_3$ | $CH_3$ | H | 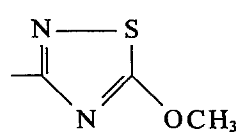 | viskoses Öl |
| 10 | $C_2H_5$ | $C_2H_5$ | H | 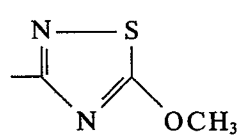 | viskoses Öl |

Fortsetzung

| Bsp. Nr. | $R^1$ | $R^2$ | $R^3$ | 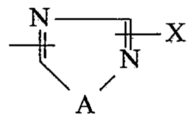 | Schmelzpunkt (°C) bzw. Brechungsindex |
|---|---|---|---|---|---|
| 11 | $CH_3$ | $C_2H_5$ | H | 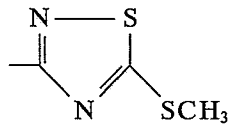 | $n_D^{20} = 1,6012$ |
| 12 | $C_2H_5$ | $C_2H_5$ | H | 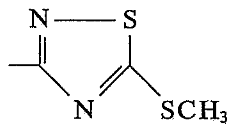 | $n_D^{20} = 1,6073$ |
| 13 | t-$C_4H_9$ | H | H | 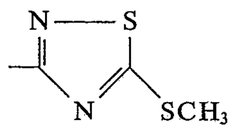 | $n_D^{20} = 1,5732$ |
| 14 | $CH_3$ | $C_2H_5$ | H | 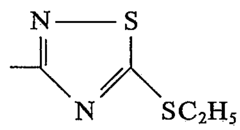 | viskoses Öl |
| 15 | $CH_3$ | $C_2H_5$ | H | 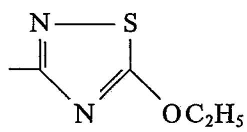 | viskoses Öl |
| 16 | $CH_3$ | $CH_3$ | H | 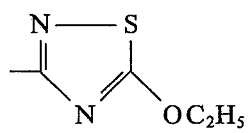 | viskoses Öl |
| 17 | $C_2H_5$ | $C_2H_5$ | H | 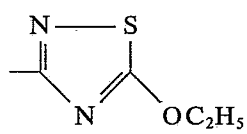 | viskoses Öl |
| 18 | t-$C_4H_9$ | H | H | 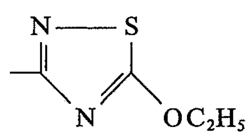 | viskoses Öl |
| 19 | $CH_3$ | $CH_3$ | H | 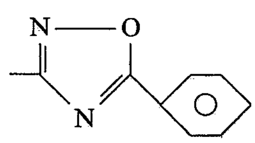 | viskoses Öl |
| 20 | $C_2H_5$ | $C_2H_5$ | H | 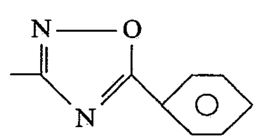 | viskoses Öl |
| 21 | t-$C_4H_9$ | H | H | 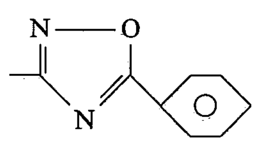 | viskoses Öl |

Fortsetzung

| Bsp. Nr. | $R^1$ | $R^2$ | $R^3$ | 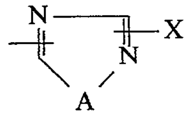 | Schmelzpunkt (°C) bzw. Brechungsindex |
|---|---|---|---|---|---|
| 22 | $CH_3$ | $CH_3$ | H | 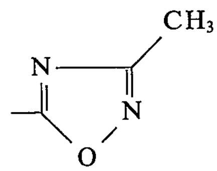 | 87–88°C |
| 23 | $CH_3$ | $CH_3$ | H | 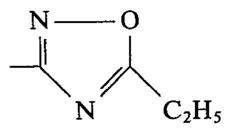 | $n_D^{20} = 1{,}5439$ |
| 24 | $CH_3$ | $C_2H_5$ | H | 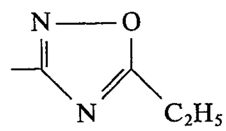 | $n_D^{20} = 1{,}5409$ |
| 25 | $C(CH_3)_3$ | H | H | 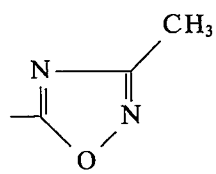 | 120–121 |
| 26 | $CH_3$ | $C_2H_5$ | H | 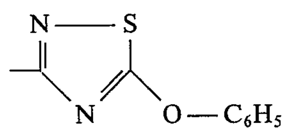 | viskoses Öl |
| 27 | $CH_3$ | $CH_3$ | H | 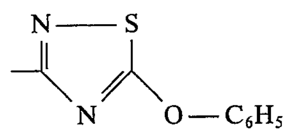 | viskoses Öl |
| 28 | $C_2H_5$ | $C_2H_5$ | H | 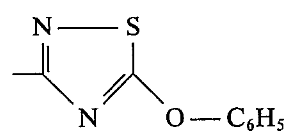 | viskoses Öl |
| 29 | $CH_3$ | $CH_3$ | H | 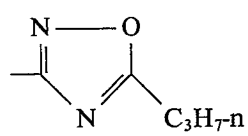 | viskoses Öl |
| 30 | $C_2H_5$ | $C_2H_5$ | H | 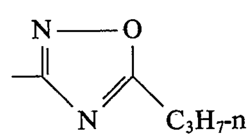 | viskoses Öl |
| 31 | $C(CH_3)_3$ | H | H | 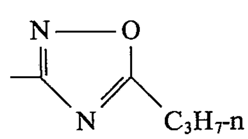 | viskoses Öl |

Fortsetzung

| Bsp. Nr. | $R^1$ | $R^2$ | $R^3$ | 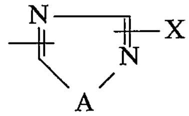 | Schmelzpunkt (°C) bzw. Brechungsindex |
|---|---|---|---|---|---|
| 32 | $C_2H_5$ | $C_2H_5$ | H | 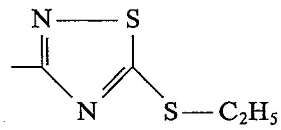 | viskoses Öl |
| 33 | $CH_3$ | $CH_3$ | H | 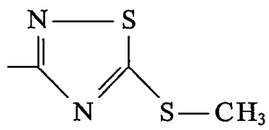 | 55 |
| 34 | $CH_3$ | $CH_3$ | H | 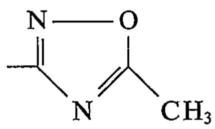 | 94 |
| 35 | $CH_3$ | H | H | 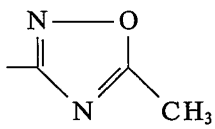 | 78 |
| 36 | $C(CH_3)_3$ | H | H | 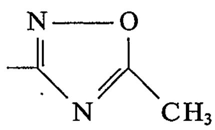 | viskoses Öl |
| 37 | $CH_3$ | H | 5-$CH_3$ | 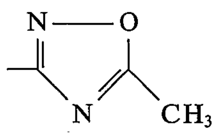 | 94 |
| 38 | $CH_3$ | H | 3-$CH_3$ | 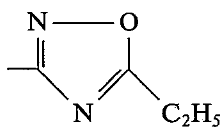 | viskoses Öl |
| 39 | $CH_3$ | H | 5-$CH_3$ | 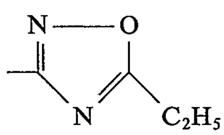 | viskoses Öl |
| 40 | $C(CH_3)_3$ | H | H | 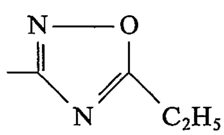 | viskoses Öl |

Nach dem in der Anmeldung beschriebenen Verfahren und entsprechend den Beispielen 1 und 2 werden die in der nachstehenden Tabelle 2 formelmäßig aufgeführten Ausgangsprodukte der Formel (II) erhalten.

Tabelle 2

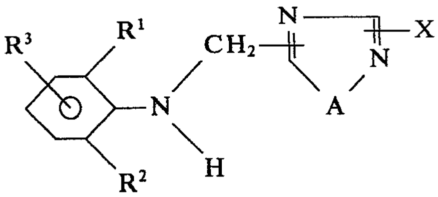

(II)

| Bsp. Nr. | $R^1$ | $R^2$ | $R^3$ | 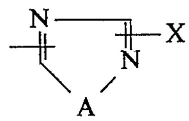 | Physikalische Konstante |
|---|---|---|---|---|---|
| (II-3) | $CH_3$ | $C_2H_5$ | H | 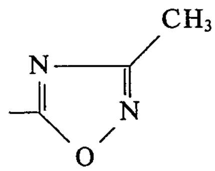 | Kp. 0,1/130–40°C |
| (II-4) | $C_2H_5$ | $C_2H_5$ | H | 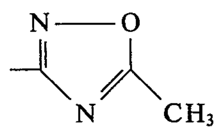 | Fp.: 78–80°C |
| (II-5) | $C_2H_5$ | $C_2H_5$ | H | 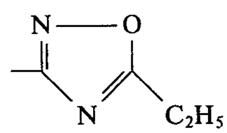 | Kp. 0,1/180–88°C |
| (II-6) | $CH_3$ | $C_2H_5$ | H | 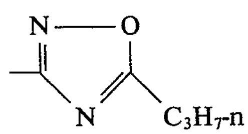 | viskoses Öl |
| (II-7) | $CH_3$ | $C_2H_5$ | H | 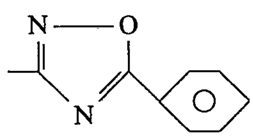 | viskoses Öl |
| (II-8) | $C_2H_5$ | $C_2H_5$ | H | 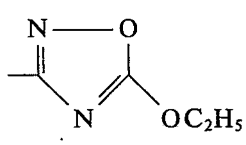 | viskoses Öl |
| (II-9) | $CH_3$ | $C_2H_5$ | H | 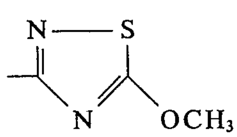 | viskoses Öl |
| (II-10) | $CH_3$ | $CH_3$ | H | 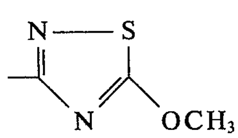 | viskoses Öl |
| (II-11) | $C_2H_5$ | $C_2H_5$ | H | 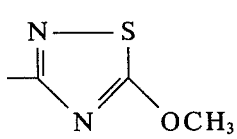 | viskoses Öl |

Fortsetzung

| Bsp. Nr. | $R^1$ | $R^2$ | $R^3$ | 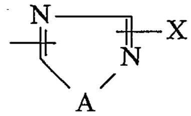 | Physikalische Konstante |
|---|---|---|---|---|---|
| (II-12) | $CH_3$ | $C_2H_5$ | H | 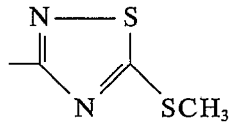 | Kp. 155–160°C/ 0,1 mm |
| (II-13) | t-$C_4H_9$ | $C_2H_5$ | H | 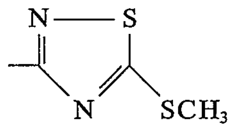 | Kp. 220°C/0,1 mm |
| (II-14) | $CH_3$ | $C_2H_5$ | H | 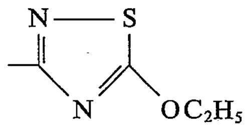 | Kp. 200°C/0,08 mm |
| (II-15) | $CH_3$ | $C_2H_5$ | H | 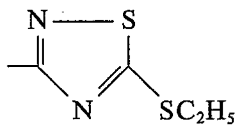 | viskoses Öl |
| (II-16) | $CH_3$ | $CH_3$ | H | 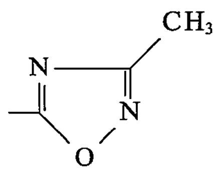 | Kp. 0,2/110–13°C |
| (II-17) | $CH_3$ | $CH_3$ | H | 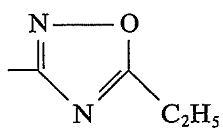 | $n_D^{20} = 1{,}5375$ |
| (II-18) | $CH_3$ | $C_2H_5$ | H | 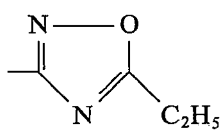 | $n_D^{20} = 1{,}5348$ |
| (II-19) | $C(CH_3)_3$ | H | H | 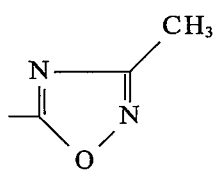 | Fp: 85–92°C |

Nach bekannten Verfahren werden die in der nachstehenden Tabelle 3 formelmäßig aufgeführten Ausgangsprodukte der Formel (IV) erhalten.

Tabelle 3

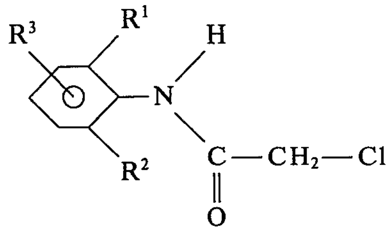

(IV)

| Bsp. Nr. | $R^1$ | $R^2$ | $R^3$ | Schmelzpunkt (°C) |
|---|---|---|---|---|
| (IV-1) | $CH_3$ | $CH_3$ | H | 148 |
| (IV-2) | $C_2H_5$ | $C_2H_5$ | H | 133 |
| (IV-3) | i-$C_3H_7$ | H | H | 79 |
| (IV-4) | tert.-$C_4H_9$ | H | H | 96 |
| (IV-5) | $C_2H_5$ | H | H | 103 |
| (IV-6) | $CH_3$ | H | H | 109 |
| (IV-7) | $CH_3$ | H | 3-$CH_3$ | 135 |
| (IV-8) | $CH_3$ | H | 5-$CH_3$ | 154 |
| (IV-9) | $CH_3$ | $CH_3$ | 4-$CH_3$ | 177 |
| (IV-10) | $C_2H_5$ | $CH_3$ | 4-$CH_3$ | 134 |
| (IV-11) | sek.-$C_4H_9$ | H | H | Öl |

**Patentansprüche**

1. N-Diazolylalkyl-chloracetanilide der Formel

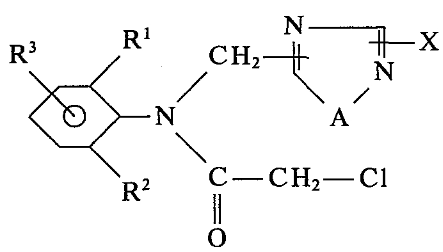

(I)

in welcher

A für Sauerstoff oder Schwefel steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
X für Wasserstoff, Alkyl mit 1 bis 4 Kohlenstoffatomen, Alkoxy mit 1 bis 4 Kohlenstoffatomen, Alkylthio mit 1 bis 4 Kohlenstoffatomen, Phenyl oder Phenoxy steht

und der Diazolyl-Rest über ein Kohlenstoffatom gebunden ist.

2. 2,6-Diethyl-N-(3′-methyl-1′,2′,4′-oxadiazol-5′-yl-methyl)-chloracetanilid der Formel

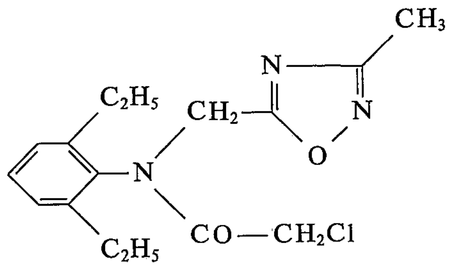

3. 2-Ethyl-6-methyl-N-(5'-methyl-1', 2', 4'-oxadiazol-3'-yl-methyl)-chloracetanilid der Formel

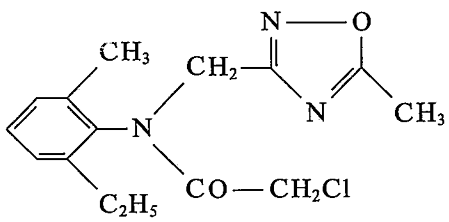

4. Verfahren zur Herstellung von N-Diazolylalkylchloracetaniliden der Formel

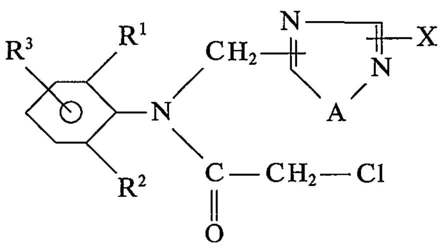

(I)

in welcher

A für Sauerstoff oder Schwefel steht,
$R^1$ für Alkyl mit 1 bis 4 Kohlenstoffatomen steht,
$R^2$ für Wasserstoff oder Alkyl mit 1 bis 4 Kohlenstoffatomen steht,
$R^3$ für Wasserstoff oder Alkyl mit 1 bis 4 Kohlenstoffatomen steht,
X für Wasserstoff, Alkyl mit 1 bis 4 Kohlenstoffatomen, Alkoxy mit 1 bis 4 Kohlenstoffatomen, Alkylthio mit 1 bis 4 Kohlenstoffatomen, Phenyl oder Phenoxy steht

und der Diazolyl-Rest über ein Kohlenstoffatom gebunden ist, dadurch gekennzeichnet, daß man

a) N-Diazolylalkyl-aniline der Formel

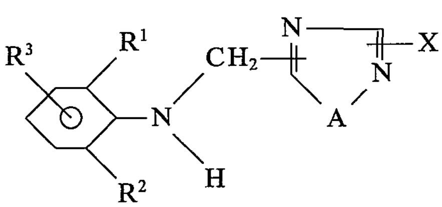

(II)

in welcher

A, $R^1$, $R^2$, $R^3$ und X die oben angegebene Bedeutung haben,

und der Diazolyl-Rest über ein Kohlenstoffatom gebunden ist, mit Chloressigsäurechlorid oder -bromid bzw. -anhydrid der Formeln

$$ClCH_2—CO—Cl(Br) \qquad (IIIa)$$

bzw.

$$(Cl—CH_2—CO)_2O \qquad (IIIb)$$

in Gegenwart eines Verdünnungsmittels und gegebenenfalls in Gegenwart eines Säurebindemittels umsetzt, oder

b) Chloracetanilide der Formel

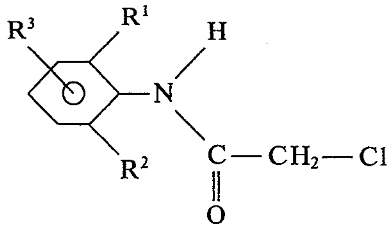

(IV)

in welcher

$R^1$, $R^2$ und $R^3$ die oben angegebene Bedeutung haben,

mit Diazolylalkyl-Derivaten der Formel

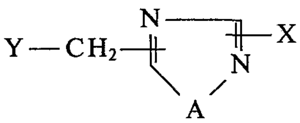

(V)

in welcher

A und X die oben angegebene Bedeutung haben und
Y für Halogen, den Mesylat- oder Tosylat-Rest steht und der

Diazolyl-Rest über ein Kohlenstoffatom gebunden ist, in Gegenwart eines Säurebinders und gegebenenfalls in Gegenwart eines organischen Verdünnungsmittels umsetzt.

5. Herbizide Mittel, gekennzeichnet durch einen Gehalt an mindestens einem N-Diazolyl-alkyl-chloracetanilid der Formel (I).

6. Verfahren zur Herstellung von herbiziden Mitteln, dadurch gekennzeichnet, daß man N-Diazolylalkylchloracetanilide der Formel (I) mit Streckmitteln und/oder oberflächenaktiven Mitteln vermischt.

7. Verwendung von N-Diazolylalkyl-chloracetaniliden der Formel (I) zur Bekämpfung von Unkräutern.

**Claims**

1. N-Diazolylalkyl-chloroacetanilides of the formula

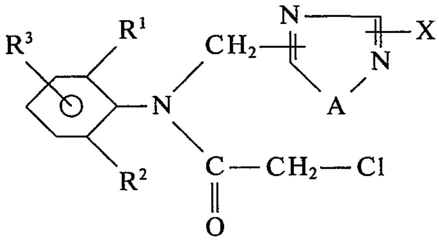

(I)

in which

A represents oxygen or sulphur,
$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
X represents hydrogen, alkyl with 1 to 4 carbon atoms, alkoxy with 1 to 4 carbon atoms, alkylthio with 1 to 4 carbon atoms, phenyl or phenoxy

and the diazolyl radical is bonded via a carbon atom.

2. 2,6-Diethyl-N-(3′-methyl-1′,2′,4′-oxadiazol-5′-yl-methyl)-chloroacetanilide of the formula

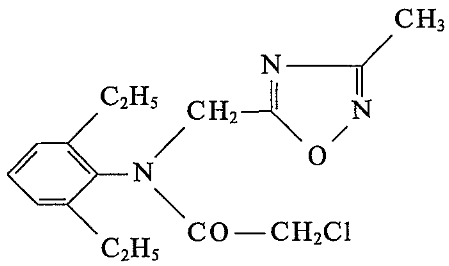

3. 2-Ethyl-6-methyl-N-(5′-methyl-1′, 2′, 4′-oxadiazol-3′-yl-methyl)-chloracetanilid of the formula

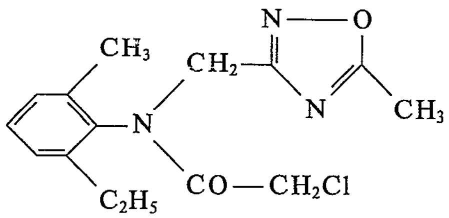

4. Process for the preparation of N-diazolylalkyl-chloroacetanilides of the formula

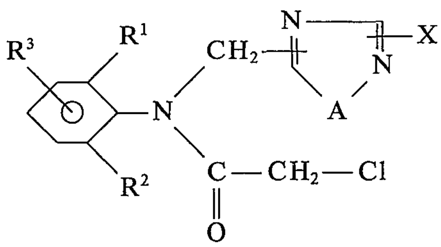

(I)

in which

A represents oxygen or sulphur,
$R^1$ represents alkyl with 1 to 4 carbon atoms,
$R^2$ represents hydrogen or alkyl with 1 to 4 carbon atoms,
$R^3$ represents hydrogen or alkyl with 1 to 4 carbon atoms,
X represents hydrogen, alkyl with 1 to 4 carbon atoms, alkoxy with 1 to 4 carbon atoms, alkylthio with 1 to 4 carbon atoms, phenyl or phenoxy

and the diazolyl radical is bonded via a carbon atom, characterised in that

a) N-diazolylalkyl-anilines of the formula

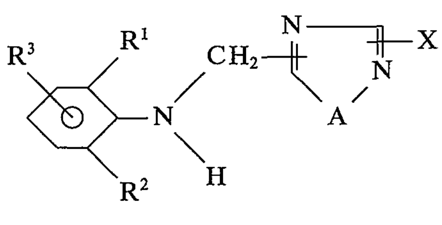

(II)

in which
A, $R^1$, $R^2$, $R^3$ and X have the meaning indicated and the diazolyl radical is bonded via a carbon atom,

are reacted with chloroacetic acid chloride or bromide or anhydride of the formulae

$$ClCH_2—CO—Cl(Br) \qquad (IIIa)$$

or

$$(Cl—CH_2—CO)_2O \qquad (IIIb)$$

in the presence of a diluent and if appropriate in the presence of an acid-binding agent, or

b) chloroacetanilides of the formula

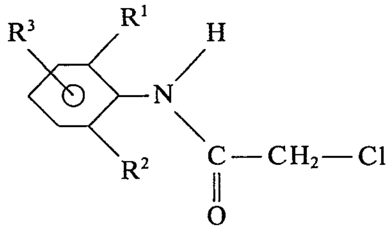

(IV)

in which
$R^1$, $R^2$ and $R^3$ have the meaning indicated above,

are reacted with diazolylalkyl derivatives of the formula

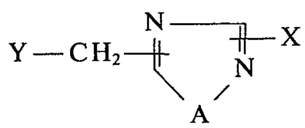

(V)

in which

A and X have the meaning indicated above and
Y represents halogen, the mesylate or tosylate radical and the diazolyl radical is bonded via a carbon atom,

in the presence of an acid binder and optionally in the presence of an organic diluent.

5. Herbicidal agents, characterised in that they contain at least one N-diazolylalkyl-chloroacetanilide of the formula (I).

6. Process for the preparation of herbicidal agents, characterised in that N-diazolylalkyl-chloroacetanilides of the formula (I) are mixed with extenders and/or surface-active agents.

7. Use of N-diazolylalkyl-chloroacetanilides of the formula (I) for combating weeds.

**Revendications**

1. N-diazolylalkyl-chloracétanilides de formule

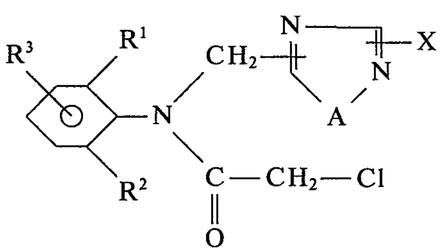

(I)

dans laquelle

A représente l'oxygène ou le soufre,
$R^1$ représente un groupe alkyle en $C_1-C_4$,
$R^2$ représente l'hydrogène ou un groupe alkyle en $C_1-C_4$,
$R^3$ représente l'hydrogène ou un groupe alkyle en $C_1-C_4$,
X représente l'hydrogène, un groupe alkyle en $C_1-C_4$, alcoxy en $C_1-C_4$, alkylthio en $C_1-C_4$, phényle ou phénoxy, et

le reste diazolyle est relié par l'intermédiaire d'un atome de carbone.

2. Le 2,6-diéthyl-N-(3'-méthyl-1',2',4'-oxadiazole-5'-yl-méthyl)-chloracétanilide de formule

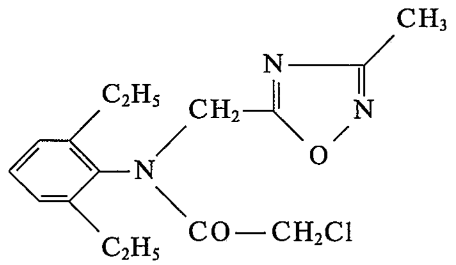

3. Le 2-éthyl-6-méthyl-N-(5'-méthyl-1', 2', 4'-oxadiazole-3'-yl-méthyl)-chloracétanilide de formule

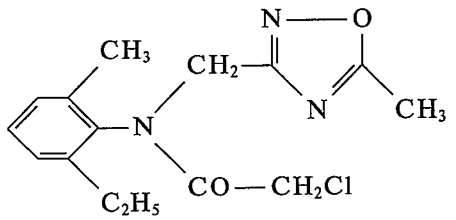

4. Procédé de préparation des N-diazolylalkyl-chloracétanilides de formule

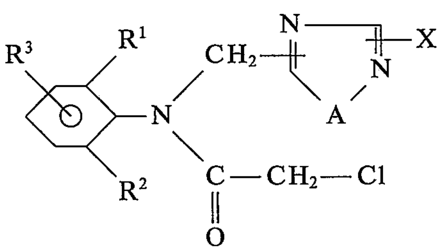

(I)

dans laquelle

A représente l'oxygène ou le soufre,
$R^1$ représente un groupe alkyle en $C_1-C_4$,
$R^2$ représente l'hydrogène ou un groupe alkyle en $C_1-C_4$,
$R^3$ représente l'hydrogène ou un groupe alkyle en $C_1-C_4$,
X représente l'hydrogène, un groupe alkyle en $C_1-C_4$, alcoxy en $C_1-C_4$, alkylthio en $C_1-C_4$, phényle ou phénoxy, et

le reste diazolyle est relié par l'intermédiaire d'un atome de carbone, caractérisé en ce que

a) on fait réagir des N-diazolylalkyl-anilines de formule

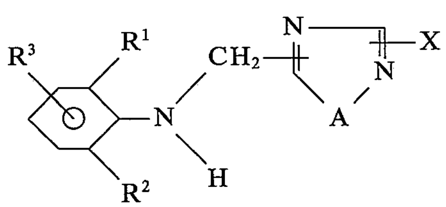

(II)

dans laquelle A, $R^1$, $R^2$, $R^3$ et X ont les signigications indiquées ci-dessus, et le reste diazolyle est relié par l'intermédiaire d'un atome de carbone, avec le chlorure, le bromure ou l'anhydride de l'acide chloracétique, répondant aux formules respectives

$$ClCH_2-CO-Cl(Br) \qquad (IIIa)$$

et

$$(Cl-CH_2-CO)_2O \qquad (IIIb)$$

en présence d'un diluant et, le cas échéant, en présence d'un agent fixant les acides, ou bien

b) on fait réagir des chloracétanilides de formule

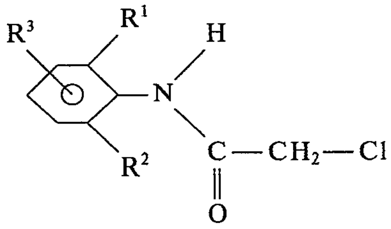

(IV)

dans laquelle $R^1$, $R^2$ et $R^3$ ont les significations indiquées ci-dessus, avec des dérivés de diazolylalkyle de formule

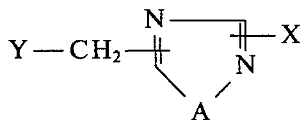

(V)

dans laquelle A et X ont les significations indiquées ci-dessus et Y représente un halogène, le reste mésylate ou tosylate et le reste diazolyle est relié par l'intermédiaire d'un atome de carbone,

en présence d'un agent fixant les acides et, le cas échéant, en présence d'un diluant organique.

5. Produit herbicide, caractérisé en ce qu'il contient au moins un N-diazolylalkyl-chloracétanilide de formule I.

6. Procédé de préparation de produits herbicides, caractérisé en ce que l'on mélange des N-diazolylalkyl-chloracétanilides de formule I avec des diluants et/ou des agents tensioactifs.

7. Utilisation des N-diazolylalkyl-chloracétanilides de formule I dans la lutte contre les mauvaises herbes.